Europäisches Patentamt

European Patent Office

Office européen des brevets

(11) Publication number: 0 467 366 A2

# EUROPEAN PATENT APPLICATION

(21) Application number: 91112033.5

(22) Date of filing: 18.07.91

(51) Int. Cl.5: **C12N 15/12**, C07K 15/06, A61K 37/02, C12P 21/08, G01N 33/574, C12Q 1/68

(30) Priority: 19.07.90 US 554466

(43) Date of publication of application:
22.01.92 Bulletin 92/04

(84) Designated Contracting States:
AT BE CH DE DK ES FR GB GR IT LI LU NL SE

(71) Applicant: THE ONTARIO CANCER INSTITUTE
500 Sherbourne Street
Toronto Ontario M4X 1K9(CA)

(72) Inventor: Ling, Victor
47 St. Clair Avenue West, Suite 1104
Toronto, Ontario M4V 1K6(CA)
Inventor: Kawai, Kazuo
51 Heath Street West
Toronto, Ontario M4V 1P2(CA)

(74) Representative: Vossius & Partner
Siebertstrasse 4 P.O. Box 86 07 67
W-8000 München 86(DE)

(54) Cell membrane glycoprotein correlated with resistance to platinum-containing antineoplastic agents.

(57) The invention relates to a novel plasma membrane glycoprotein having an approximate molecular weight of 200 kDa. The glycoprotein is correlated with CDDP-resistance in murine thymic lymphoma cell sublines from which it was isolated. The glycoprotein, designated CP$^R$-200, appears to be correlated with resistance to the platinum family of anti-neoplastic drugs across species.

EP 0 467 366 A2

The invention relates to a novel plasma membrane glycoprotein correlated with resistance to platinum-containing antineoplastic agents, particularly cis-diamminedichloroplatinum (II) (CDDP), as observed in a series of murine thymic lymphoma cell sublines. The membrane glycoprotein, which has been designated CP[R]-200, has been observed in cell lines from other species including human, and is associated with reduced platinum drug accumulation in cancer cells.

Platinum-containing antineoplastic agents such as CDDP or analogs carboplatin, tetraplatin and iproplatin have found wide use in the treatment of certain types of cancer. In particular, CDDP has become one of the most useful anti-neoplastic agents in cancer chemotherapy. Chemotherapy with CDDP has excellent initial effectiveness against various malignant tumors such as testicular, ovarian, bladder, and head and neck carcinomas. It is generally believed, however, that the development of CDDP-resistant cells in residual tumors results in the eventual failure of chemotherapy in many patients. A number of studies have described the isolation of CDDP-resistant cells in vitro, and have postulated different mechanisms for the resistance toward CDDP. These mechanisms include an increase in metallothionein and glutathione as well as an accelerated DNA repair system.

The selection of a series of CDDP-resistant variants from a murine lymphoma cell line R1.1 which display a decrease in cellular drug accumulation correlated with their degree of drug resistance has been recently described (Kawai et al. (1988) Mie Med. J. 38, 273-281). Similar to other drug accumulation altered mutants, such as multidrug resistant (MDR) cells, it was reasoned that this class of CDDP-resistant cells may also involve a plasma membrane alteration.

Mechanisms of resistance to CDDP have been investigated using cell lines selected in vitro. Some postulated mechanisms have included an increase in metallothionein, the intracellular heavy metal-detoxifying protein, or glutathione, which might inactivate the drug, as well as an accelerated DNA repair system. Recently, reduced intracellular drug accumulation has also been proposed as a possible mechanism. The present work describes the characterization of a series of CDDP-resistant murine lymphoma lines which display a reduced ability to accumulate CDDP. As described in detail elsewhere, the level of CDDP-resistance has been shown to correlate with decreased intracellular drug accumulation (Kawai et al (1988)). It is not known at this point whether or not this reduced CDDP accumulation is the sole cause of CDDP-resistance in this series of resistant lymphoma lines. It has been suggested that a number of factors may contribute to a cell's overall resistance to CDDP; however, the objective of this study was to determine if a molecular difference could be identified in the plasma membranes of CDDP-resistant cells compared to the sensitive parental cells. Since the study of the plasma membranes of accumulation defective MDR cells resulted in the identification of the presumptive drug-efflux pump protein, P-glycoprotein, it was reasoned that a similar approach might lead to membrane components involved in the transport of CDDP.

Thus, the technical problem underlying the present invention is to provide a membrane protein which may be associated with increased CDDP resistance. Further technical problems are to provide DNA sequences encoding the protein or parts thereof and monoclonal antibodies directed to the protein or parts thereof.

The solution to these technical problems is achieved by providing the embodiments characterized in the claims.

Accordingly, the invention provides an isolated plasma membrane antigen of approximately 200 kDa (CP[R]-200) that was initially characterized from an overexpression in murine lymphoma sublines selected for resistance to CDDP. The expression of CP[R]-200 appears to correlate with an increase in the degree of resistance. This is the first identification of a plasma membrane component associated with CDDP-resistance. The membrane component appears to play a role in the reduced accumulation of CDDP observed in this class of CDDP-resistant cells. It is reasonable to expect that the expression of CP[R]-200 is correlated generally with resistance to platinum-containing antineoplastic agents.

The invention also includes nucleotide sequences, such as cDNA sequences, which encode the antigen or portions of the antigen. Such nucleotide sequences have a variety of uses including use as probes for diagnostic and research purposes. Isolation of the antigen CP[R]-200 affords the ability to derive monoclonal antibodies, which are also within the scope of the invention.

Figure 1 is a schematic representation of the derivation of CDDP-resistant sublines used in the isolation of the glycoprotein of the invention.

Figure 2 are graphs showing dose responses of the drug-sensitive parental line R1.1 and three CDDP-resistant cell lines, where

O—O , R1.1 cells; ●—● , E8 cells; △—△ , E5 cells; ▲—▲ ,B8 cells.

2

Figures 3 A & B show the immunological detection of CDDP-resistance associated cell membrane protein by Western blots.

Figure 4 shows the immunological detection of P-glycoprotein in CDDP-resistant cells by Western blot.

Figure 5 shows the immunological detection of 200 kDa CDDP-resistance associated membrane protein $CP^R$-200 by Western blot.

Figure 6 shows the Western blot of proteins subjected to deglycosylation as compared to the same proteins not so treated.

Figure 7A shows the subcellular localization of $CP^R$-200 by Western blot, and Figure 7B shows an immunofluorescence staining of E5 resistant cells showing that $CP^R$-200 is a membrane protein.

One object of the present invention relates to a plasma membrane antigen having a molecular weight of approximately 200kDa and being correlated with resistance to platinum-containing anti-neoplastic agents.

Another object of the present invention relates to a monoclonal antibody specific to a plasma membrane antigen having a molecular weight of approximately 200kDa and being correlated with resistance to platinum-containing anti-neoplastic agents.

Another object of the present invention relates to a nucleotide sequence encoding a plasma membrane antigen having a molecular weight of approximately 200kDa and being correlated with resistance to platinum-containing anti-neoplastic agents or fragments thereof.

Another object of the present invention relates to a nucleotide sequence capable of hybridizing to a nucleotide sequence encoding a plasma membrane antigen having a molecular weight of approximately 200kDa and being correlated with resistance to platinum-containing anti-neoplastic agents or fragments thereof.

Another object of the present invention relates to an expression vector comprising a nucleotide sequence encoding a plasma membrane antigen having a molecular weight of approximately 200kDa and being correlated with resistance to platinum-containing anti-neoplastic agents or fragments thereof.

Another object of the present invention relates to a cell containing a functional expression vector capable of replication in the cell, the vector comprising transcriptional and translational signals functional in the cell and a DNA sequence encoding a plasma membrane antigen having a molecular weight of approximately 200kDa and being correlated with resistance to platinum-containing anti-neoplastic agents or fragment thereof and being under the transcriptional and translational control of said signals.

Another object of the present invention relates to the use of said monoclonal antibody or said nucleotide sequences for detection and diagnosis of cells resistant to platinum-containing anti-neoplastic agents.

Another object of the present invention relates to a composition or a kit containing at least one monoclonal antibody of the present invention or at least one nucleotide sequence of the present invention for detection and diagnosis of cells resistant to platinum-containing anti-neoplastic agents.

The fact that $AS_{E5}$ detects $CP^R$-200 faintly but regularly in plasma membranes from sensitive cells suggests that this component is already expressed in the parental line. Therefore, it is likely that $CP^R$-200 is a component synthesized by CDDP-resistant cells in increased levels.

This invention provides the initial characterization of $CP^R$-200. It has been demonstrated that $CP^R$-200 is unlikely a form of P-glycoprotein since CDDP-resistant cells are not cross-resistant to two commonly associated MDR drugs, adriamycin and vinblastine. Moreover, Western blot analyses using the P-glycoprotein specific monoclonal antibody C219 did not reveal a change in the expression of P-glycoprotein in the CDDP-resistant cells. Also, $CP^R$-200 is not related to the 190 kDa protein detected by $AS_{R1.1}$ antiserum. The 190 kDa protein detected by the antiserum $AS_{R1.1}$ may be the L-CA. The phenotypic and functional changes of L-CA within individual T cell subsets during T cell ontogeny as well as in B cells are well documented; thymocytes express the lowest apparent molecular weight form of 180 kDa, B lymphocytes express the highest form, 220 kDa, and T lymphocytes express multiple forms. The question arises whether the CDDP-resistant sublines have undergone differentiation to more mature subsets during the acquisition of CDDP-resistance in vitro and whether the membrane glycoprotein specifically detected by $AS_{E5}$ might belong to the isoforms of L-CA. This is unlikely because examination of well-known T cell differentiation markers, Ly2 and L3T4 indicate that the resistant cells are still phenotypically equivalent to double negative thymocytes. According to the data reported by Lefracois et al. (1987) J. Immunol. 139, 3718-3724, thymocytes bearing such phenotypes should essentially express only the lowest molecular weight isoform of L-CA. Furthermore, if the antiserum $AS_{R1.1}$ detects L-CA as anticipated, it should recognize all isoforms of L-CA similar to the rabbit antiserum raised against mouse lymphocytes described previously.

The precise function of $CP^R$-200 is presently unknown. Whether this membrane antigen over-expressed in drug-resistant cells is, in fact, the causative membrane protein mediating decreased drug accumulation or merely the by-product of some as yet unknown mechanism, is not clear. It may be that analogous to the membrane P-glycoprotein associated with multidrug resistance, this surface glycoprotein is involved in mediating the reduced accumulation of CDDP in resistant cells.

While the foregoing description has related particularly to the correlation of $CP^R$-200 with CDDP-resistant cells, the skilled person will appreciate the isolated membrane antigen is correlated generally with resistance to platinum-containing antineoplastic agents. For example, platinum-containing CDDP analogs such as carboplatin, tetraplatin or iproplatin appear to be involved in cross resistance to CDDP resistant cells, so $CP^R$-200 would also be correlated with such cross resistance.

Nucleotide sequences encoding all or portions of $CP^R$-200 may be obtained using the standard λgt11 cloning method in conjunction with detection using polyclonal or monoclonal antibody against $CP^R$-200 (see, for example, Riordan et al., 1985, Nature 316: 817-819). Such sequences may be cDNA sequences which may have use as probes against $CP^R$-200 for the purpose of detection and diagnosis of cells resistant to platinum-containing antineoplastic agents. In this regard the invention includes nucleotide sequences having at least 60% homology with a sequence encoding all or a portion of $CP^R$-200 over a span of at least 50 nucleotide bases as measured by hybridization therewith.

The skilled person will appreciate that the invention also includes hybrid DNA to effect expression of all or a portion of $CP^R$-200 in a cell system. Thus, a DNA sequence of the invention may be joined to a non-wild type DNA to form a hybrid DNA, wherein the non-wild type DNA comprises a replication system recognized by a unicellular microorganism. Expression vectors comprising a nucleotide sequence of the invention are also within the scope of the invention. Such vectors may afford expression of $CP^R$-200 or a fragment in either prokaryotic or eukaryotic cells.

Monoclonal and serum antibodies may be raised against $CP^R$-200 or epitopic portions thereof using standard methods (see, for example, Kartner et al., 1985, Nature 316: 820-823). Such antibodies may be useful for detection and diagnosis of cells resistant to platinum-containing antineoplastic agents.

Accordingly, such serum and monoclonal antibodies are included within the scope of the invention.

Selection and Cellular Properties of CDDP-Resistant Sublines

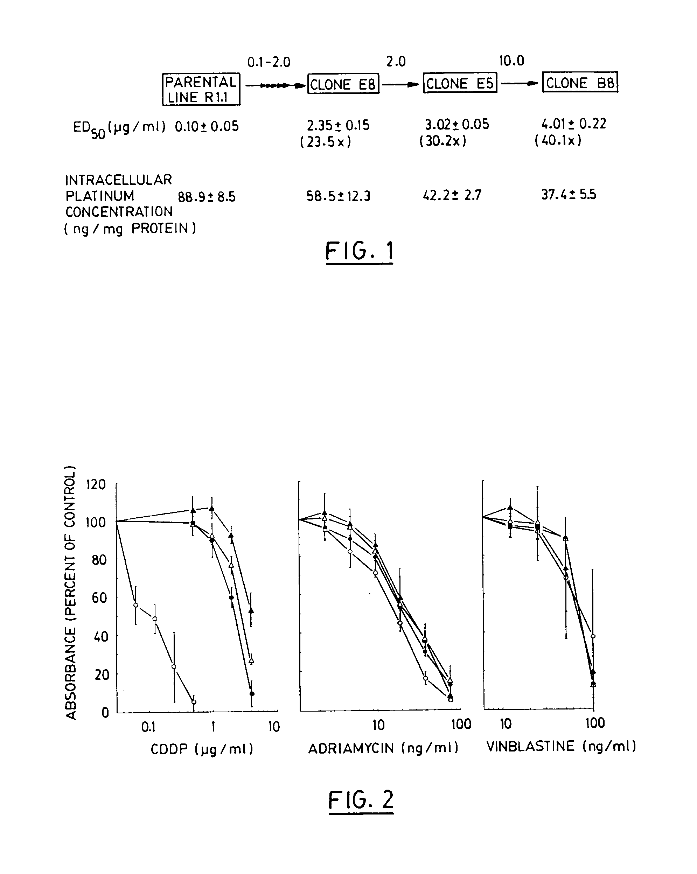

CDDP-resistant sublines were selected in vitro by continuous exposure of drug-sensitive R1.1 murine lymphoma cells to increasing amounts of CDDP. Figure 1 shows an outline of the selection procedure used to isolate the different cell variants. The first step of selection involved an incremental increase in CDDP concentration from 0.1 to 2.0 μg/ml over a period of 9 months. The resistant clone R1.1/CDDPR-E8 (E8) was about 20-fold more resistant than parental cells as demonstrated by their $ED_{50}$ values (Fig. 1). The next resistant variant cells, R1.1/CDDPR-E8/E5 (E5), were sequentially isolated from E8 in the presence of 2.0 μg/ml of CDDP continuously for 6 months. The third step selectants were obtained from E5 after 20 cycles of intermittent exposure (10 μg/ml of CDDP). Clones were obtained at each step by limiting dilution.

The degree of CDDP-resistance was previously shown to correlate with the reduction in CDDP accumulation in vitro. It is noteworthy that there was a rank correlation in the cellular concentration of accumulated platinum and the increased resistance to CDDP in this series of lines (Fig. 1). To evaluate the drug resistance properties of CDDP-resistant clones, their chemosensitivities to various cytotoxic drugs were examined using the growth inhibition MTT assay. Fig. 2 shows the effect of increasing cytotoxic drug concentration on the growth of parental R1.1 and the resistant sublines. The resistance of cells towards CDDP increased with each selection step, whereas two commonly used antineoplastic agents, adriamycin and vinblastine, were equally cytotoxic to both the resistant and parental lines. Each point represents the percentage of absorbance values obtained from 3 separate assays in comparison to those in drug-free culture. Thus, it appears that the CDDP-resistant phenotype in these cell lines is different from the MDR phenotype, and thus, may be limited to CDDP and related analogues.

Interestingly, CDDP-resistant cells were found to become non-tumorigenic in syngeneic C58 mice from which R1.1 cells were originally derived; none of the mice inoculated with E5 cells developed any tumors within the four-week observation period after the transplantation. However, all mice inoculated with R1.1 cells developed ascitic tumors as well as subcutaneous tumors located at the injection sites around 10 days after inoculation. Similar observations have been noted in other membrane altered drug-resistant cells, most notably the MDR-hamster cells. The basis for decreased tumorigenic activity is not known, but possibly it may involve changes in the expression of particular cell surface antigens.

Immunological Characterization

It was previously demonstrated that the degree of CDDP-resistance in the cell lines described here correlated with the reduction in drug accumulation, although it was not determined if a membrane alteration was associated with CDDP-resistance. To identify such alteration, a comparison of the profile of the membrane proteins derived from the CDDP-resistant sublines as well as the parental cell line was undertaken. The initial characterization using SDS-PAGE of the plasma membrane of sensitive and resistant cells did not reveal any apparent differences in the composition of the plasma membrane components stained by Coomassie brilliant blue.

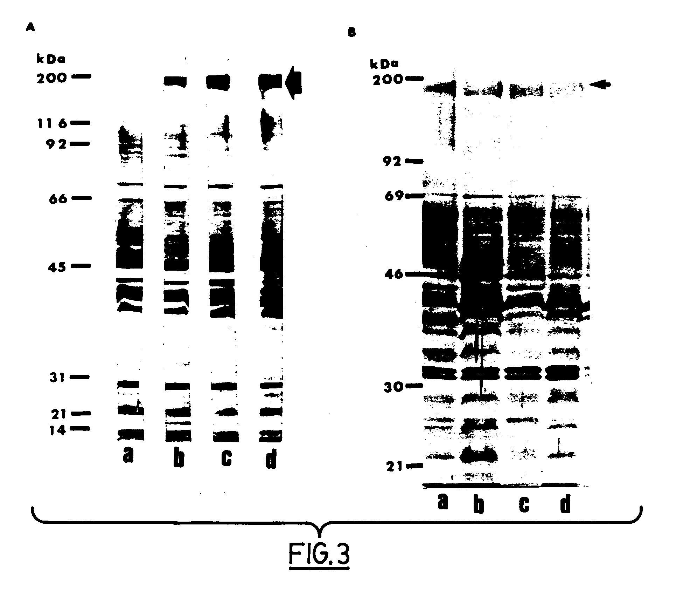

However, antigenic differences were detected by an immunological approach using antisera raised in rabbits against plasma membrane of the resistant cells. Analysis of the plasma membranes from parental and resistant cells by Western blot analysis revealed a major difference in the profile of the membrane fractions. Fig. 3A shows the results of a Western blot containing the different membrane fractions (a - d) probed with anti-resistant serum ($AS_{E5}$). Lane a is the sensitive parental line, R1.1; lane b is the low grade CDDP-resistant line, E8; lane c is E5; and lane d is the resistant line B8. $AS_{E5}$ serum reacts with a 200 kDa component that is overexpressed in CDDP-resistant clones. Moreover, the overexpression of this 200 kDa antigen correlates with the level of resistance to CDDP in these clones. In contrast, other membrane proteins also recognized by this antiserum were similar in both resistant and parental cells (Fig. 3A).

Since an immunological analysis using $AS_{E5}$ serum revealed a difference between the sensitive and resistant cells, antibodies were also raised against the plasma membrane from drug sensitive cells, R1.1 ($AS_{R1.1}$) to determine if there had been a loss of a membrane component in the resistant cells that was not detected by $AS_{E5}$ antibodies. Fig. 3B shows the results of a Western blot analysis using the $AS_{R1.1}$ antiserum. No detectable differences were observed in the membrane protein fractions from parental and resistant cells. However, an antigenic membrane protein of 190 kDa was detected by this antiserum. This protein may be the leukocyte common antigen (L-CA), a well known glycoprotein of approximately 200 kDa expressed on the surface of all leukocytes and their hematopoietic progeniters (Thomas (1989) Ann. Rev. Immunol. 7, 339-369).

To determine the differentiation levels of the drug-resistant cells, these cells were examined for the expression of the differentiation marker antigens, Lyt2, L3T4 and Thy 1.2. All of these cells exhibited membrane fluorescence for Thy 1.2, while they were negative for both Lyt2 and L3T4. Thus, they appear to retain the phenotype of immature thymocytes (double negative cells).

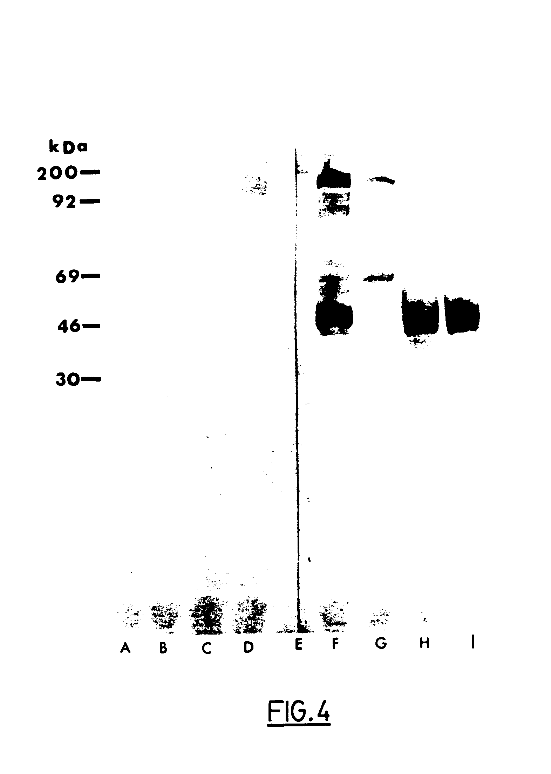

The lack of cross-resistance to unrelated drugs displayed by CDDP-resistant cells (Fig. 2) suggested that these cells were different from MDR cells. In order to rule out the possibility that the membrane alteration in CDDP-resistant cells was due to an overexpression of a modified form of P-glycoprotein, membrane fractions from R1.1 and CDDP-resistant cells (E5) were analyzed for the presence of P-glycoprotein using a P-glycoprotein-specific monoclonal antibody, C219. As shown in Fig. 4, P-glycoprotein was not detected in R1.1, E5, and LTA cells (Lanes A, B and C), while only $ECH^R$ cells (Lane D) show the presence of the 170 kDa P-glycoprotein band (Lane E contains molecular markers). Similarly, when a duplicate Western blot was probed with $AS_{E5}$, no staining of a 200 kDa protein was seen in LTA and $ECH^R$ cells (Fig. 4, Lanes H and I). Lane F is E8/E5, and Lane G is R1.1. These results suggest that the 200 kDa membrane component detected by $AS_{E5}$ antiserum in CDDP-resistant cells is different from the previously characterized P-glycoprotein.

Characterization of the CDDP-Resistance Associated 200 kDa Membrane Protein ($CP^R$-200)

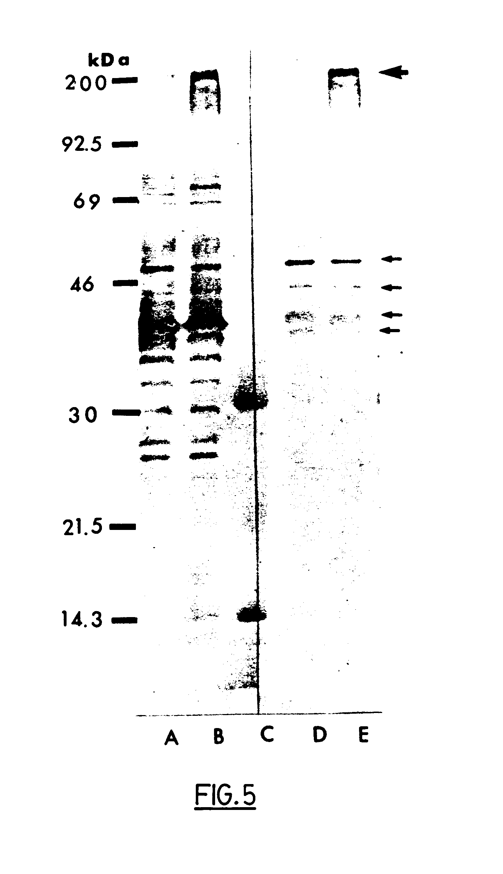

As a further step toward determining if the 200 kDa component detected at an increased level in the CDDP-resistant lines has an antigenetically related counterpart in the R1.1 sensitive cells, the antiserum $AS_{E5}$ was absorbed with membrane proteins from R1.1 cells. The absorbed serum was then used for Western blot analysis as shown in Fig. 5. Lanes A and D are R1.1; Lanes B and E are E5; and Lane C is molecular markers. The left half of the blot was overlaid with $AS_{E5}$, and the right half with the absorbed $AS_{E5}$. It was found that the number of bands detected by the absorbed $AS_{E5}$ serum is greatly reduced, and that significantly, the only detectable difference between the sensitive R1.1 cells and the resistant E5 cells is the 200 kDa component, which stained as a diffuse band. The four bands detected by this absorbed antiserum between 30 and 50 kDa are faintly stained and are present in both lines in equal amounts. It was concluded from these results that the 200 kDa component detected by the absorbed $AS_{E5}$ antiserum is an overproduced component synthesized by CDDP-resistant cells rather than the result of a post-translational processing event of a protein that is equally synthesized in sensitive R1.1 cells. This component has been designated $CP^R$-200 for its apparent association with CDDP-resistance. It is possible that $CP^R$-200 may be expressed at a low level in the drug sensitive R1.1 cells.

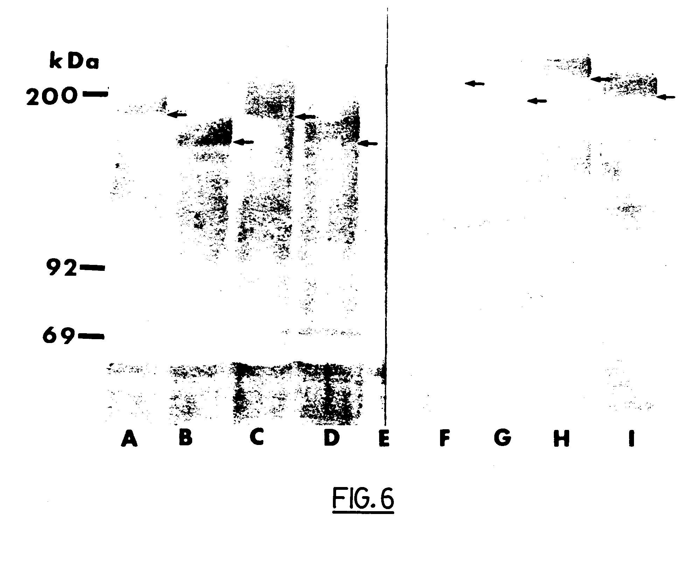

To determine if $CP^R$-200 is a glycoprotein, membrane proteins from R1.1 and E5 cells were treated with an endoglycosidase, PNGase F. This enzyme cleaves N-glycans between asparagine and the carbohydrate chain removing the bulk of the sugar moiety en bloc. Figure 6 shows the results of deglycosylation treatment of CDDP-resistance associated membrane protein. 100 $\mu$g of purified plasma membrane from the CDDP-sensitive parental line R1.1, and the CDDP-resistant subline E5 were digested with PNGase F and divided into two equal parts. SDS-PAGE and immunoblot analyses were performed and the membrane was cut into two pieces. Lanes A and F are non-deglycosylated R1.1. Lanes B and G are deglycosylated R1.1. Lanes C and H are nondeglycosylated E5. Lanes D and I are deglycosylated E5, and Lane E is molecular markers. The left half, Lanes A-D, was overlaid with $AS_{R1.1}$, and the right half, Lanes F-I, was stained with $AS_{E5.}$ As shown in Lanes H and I of Fig. 6, the apparent molecular size of $CP^R$-200 decreased from 209 kDa to 200 kDa as a result of PNGase F treatment, consistent with $CP^R$-200 being a glycoprotein. A similar shift was seen in a faintly stained band in the drug-sensitive R1.1 cells (Lanes F and G of Fig. 6) suggesting that $CP^R$-200 is likely expressed at a low level in these cells. It is not known if the PNGase F treated $CP^R$-200 is completely deglycosylated, although more extensive treatment with this endoglycosidase did not result in a further shift in the apparent molecular size of this component. The high molecular weight component detected by the $AS_{R1.1}$ antiserum in both the sensitive and resistant cells also underwent a molecular weight shift when treated with PNGase F (Lanes A-D of Fig. 6). The treated component migrated with an apparent molecular size of 162 kDa and this is clearly different from the deglycosylated form of $CP^R$-200. These findings however are consistent with the notion that the high molecular weight component detected by $AS_{R1.1}$ is likely the leukocyte-common antigen (L-CA) which has been identified as a major glycoprotein of lymphoid cells.

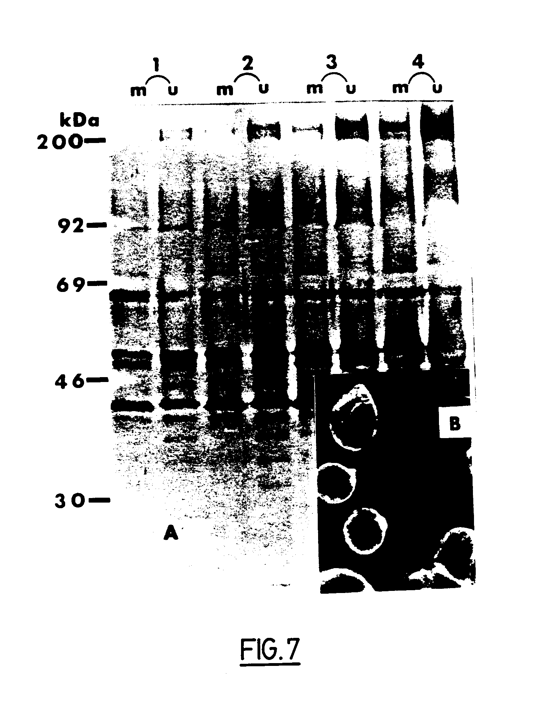

To determine the subcellular localization of $CP^R$-200 in the CDDP-resistant cells, membrane fractions from E5 cells fractionated on a discontinuous sucrose gradient were examined. A biochemical assay for the 5'-nucleotidase which is generally used to assess the level of plasma membrane purity was performed. The result of this assay (data not shown) indicated that the upper interface was enriched for plasma membrane (approximately 10-fold) compared with vesicles from the middle interface. 5'-nucleotidase activity was not detected in the lower interface. The membrane proteins from the different interfaces of the sucrose gradient were examined for the presence of $CP^R$-200 protein by Western blot analysis using $AS_{E5}$ antiserum. Figure 7A (Lanes 1-4) shows an immunoblot of the two membrane fractions obtained from the upper (u) and middle (m) interfaces of the sucrose gradient. The immunostaining for $CP^R$-200 with $AS_{E5}$ antiserum in the upper interface was at least 5-folds greater than that of the middle interface. In a separate experiment, the amount of $CP^R$-200 present in the lower interface was determined to be even less than that observed in the middle interface. All these results indicate that $CP^R$-200 is localized to the plasma membrane. Further evidence for the subcellular localization of $CP^R$-200 protein to the cell surface membrane has been obtained from immunocytochemical studies of E5 permealized cells. Figure 7B shows immunofluorescence staining of E5 cells using preabsorbed $AS_{E5}$ was predominantly localized to the cell periphery. This is consistent with $CP^R$-200 being a plasma membrane glycoprotein.

The present invention will now be described in detail in the following Examples, which should not be taken as limiting the scope of the present invention.

## 1. Cells and Culture Conditions

R1.1 and the CDDP-resistant variant cells were grown in RPMI 1640 medium supplemented with 10% fetal bovine serum (FBS), 100 units/ml penicillin, and 100 $\mu$g/ml streptomycin sulfate. LTA and $ECH^R$ cells were cultured in $\alpha$-MEM medium instead of RPMI 1640 medium. LTA is an adenine phosphoribosyl transferase-deficient mouse fibroblast cell line derived from $LMTK^-$ cells, and $ECH^R$ is a colchicine-resistant mutant isolated from an ethyl methanesulfonate-treated $LMTK^-$ culture (Debenham et al. (1982) Mol. Cell. Biol. 2, 881-889). All cell cultures were incubated at 37˚C in a humidified atmosphere containing 95% air and 5% $CO_2$. The selection protocol used to obtain the series of variant clones is illustrated in Figure 1 and has been described elsewhere in more detail (Kawai et al. supra).

## 2. Drug Resistance

The level of resistance toward CDDP, adriamycin and vinblastine was examined using the MTT assay with the slight modifications described by Carmichael et al. (1987) Cancer Res. 47, 936-942. Briefly, cell suspensions were obtained by mechanical disaggregation after brief trypsinization. Cells were cultured for 4 days in 96-well culture plates (Linbro/Titertek, Flow Laboratories, McLean, Va.) containing 180 $\mu$l of medium and 20 $\mu$l of phosphate buffered saline (PBS) or 20 $\mu$l of various concentrations of the drug to be tested in PBS at an initial plating cell density of 2.5 x $10^4$ cells/well. In the case of CDDP, the drug was dissolved in normal saline instead of PBS. Cell densities were determined using a hemocytometer and by counting viable cells in 0.1% trypan blue. The incubation time and the initial cell density were adjusted so that untreated cells were in the exponential growth phase at the time of the evaluation. For the MTT assay, 0.1 mg of 3(4,5-dimethyl thiazolyl 1-2) 2,5-diphenyl tetrazolium bromide (MTT, ICN Biochemicals, Cleveland, OH) (50 $\mu$l of 2 mg/ml in PBS) was added to each well, incubated at 37$^\circ$ C for an additional 4 h., and the plates centrifuged at 450 x g for 5 min. in a plate holder. All the medium was aspirated from each well, to which 50 $\mu$l of dimethylsulfoxide was added and the plates were placed on a plate shaker for 5 min. Absorbance at a wavelength of 540 nm was estimated using an enzyme-linked immunosorbent assay reader (Microplate autoreader EL310, Biotek Instruments, Inc., Burlington, VT). Results are expressed as an average from data of 6 wells. Fractional absorbance was calculated using the following formula: (mean absorbance of six test wells) - (mean absorbance of six reference wells) / (mean absorbance of six control wells) - (mean absorbance of six reference wells). In the control wells, cells were incubated in the absence of drug; the reference wells were treated in the same way as the control wells except no cells were added to these wells. The $ED_{50}$ values of each population were also calculated from the data obtained. $ED_{50}$ was expressed as the dose of the drug examined that inhibited the absorbance by 50%.

3. Tumorigenesis Experiments

Female C58 mice at the age of 10 to 14 weeks were used for these experiments. Ten mice were inoculated intraperitoneally with 2 x $10^6$ R1.1 or E5 cells washed in sterile PBS three times. The cells used were maintained in culture in the absence of drugs for more than two months prior to inoculation.

4. Plasma Membrane Isolation

Plasma membrane was isolated according to the method described by Riordan and Ling (1979) J. Biol. Chem. 254, 12701-12705, with some modifications. Cells were washed three times with ice-cold PBS, and the cell pellet resuspended in hypotonic lysis buffer (10 mM KC1, 1.5 mM MgC1$_2$, 2 mM phenylmethylsulfonyl fluoride (PMSF), 10 mM Tris-HC1, pH 7.4) and incubated for 30 min. A Ten Broeck homogenizer (Corning Laboratory Sciences Co.) was used to rupture the cells, and the homogenates were centrifuged at 4,000 x g for 10 min. in a Sorvall RC-5B centrifuge. Pellets enriched for plasma membranes were obtained by high-speed centrifugation at 100,000 x g for 60 min. in a Beckman SW 28 rotor. The resultant plasma membrane pellet was resuspended in a small volume of ice-cold PBS and applied to a discontinuous sucrose gradient consisting of 7 ml of 60% (w/v), 10 ml of 45%, 14 ml of 31%, and 7 ml of 16% sucrose in 5 mM Tris-HC1, pH 7.4. Centrifugation was performed in a SW 28 rotor at 100,000 x g(max) for 18 h. at 4$^\circ$ C. Materials banding at the three interfaces were recovered separately. These specimens were diluted in PBS and concentrated by centrifugation at 100,000 x g for 60 min. To assess which interface was enriched for plasma membrane, 5'-nucleotidase activity for each fraction was examined using a thin layer chromatography procedure in which AMP was used as the substrate, and adenosine formation was determined.

5. Protein Determination

The protein concentration in each sample was determined according to the method described by Lowry et al. (1951) J. Biol. Chem. 193, 265-275, using bovine serum albumin (BSA) (Fraction V, Sigma) as a standard.

6. Antisera Preparation

Cell membrane extracts were prepared from E5 and R1.1 cells as described above. Extracts containing 100 $\mu$g of protein in 1 ml of PBS were mixed with an equal volume of complete Freund's adjuvant and emulsified vigorously. Rabbits were injected with either 4 or 6 doses of the emulsion at intervals of 14 and 7 days, respectively, and then bled 10 days after the last immunization. The antisera against E5 and R1.1 were designated $AS_{E5}$ and $AS_{R1.1}$ respectively. To improve specificity, $AS_{E5}$ was absorbed with SDS-solubilized plasma membrane proteins extracted from drug-sensitive R1.1 cells bound to nitrocellulose powder. A more detailed description of this absorption method has been reported elsewhere (Kartner et al. (1983) Cancer Res. 43, 4413-4419).

7. Gel Electrophoresis and Immunostaining

Polyacrylamide slab gel electrophoresis in the presence of sodium dodecyl sulfate (SDS-PAGE) was carried out in a 1.5-mm-thick slab gel consisting of a 7% or 10% acrylamide separation gel and a 4% acrylamide stacking gel using the discontinuous buffer system of Laemmli. Samples containing 50 $\mu$g of membrane protein were suspended in SDS and boiled for 5 min. before loading onto the stacking gel. For the assessment of P-glycoprotein expression, SDS-PAGE was performed at a constant power of 5 W/gel using a modification of Fairbanks' technique as described previously (Debenham et al. supra). Molecular weights were estimated by the method of Weber and Osborn (1969) J. Biol. Chem. 244, 4406-4412, using standard proteins. After electrophoresis, protein from the gel was electrotransferred onto nitrocellulose paper (BA-85; 0.45 $\mu$m pore size, Schleicher and Schnell, Keene, NH) using the method described by Towbin et al. (1979) Proc. Natl. Acad. Sci. USA 76, 4350-4354. The replica blotted paper was incubated in 3% BSA in TBS buffer (10mM Tris - HC1, pH 7.4, 0.9% NaC1) for 2 h. at 37°C, and then reacted with 1% antiserum or 0.1% monoclonal antibody (C219) / 1% BSA in TBS buffer for 18 h. at 4°C. The paper was then immersed in 0.05% Tween 20, 500 mM NaC1, 20 mM Tris-HC1, pH 7.4 for 30 min., and washed in 500 mM NaC1, 20 mM Tris-HC1, pH 7.4 for 10 min. Peroxidase conjugated goat anti-rabbit (BRL, Gaithersburg, Md.) or anti-mouse (Sigma) immunoglobulins were mixed with 1000 volumes of 3% BSA in TBS. The paper was incubated in this mixture for 30 min., and then washed in 0.05% Tween 20, 500 mM NaC1, 20 mM Tris-HC1, pH 7.4 for 30 min. in 500 mM NaC1, 20 mM Tris-HC1, pH 7.4 for 10 min. and in 50 mM Tris-HC1, pH 7.4 for 5 min. The peroxidase reaction was performed using 3,3'-diaminobenzidine tetrahydrochloride (Sigma) and $H_2O_2$ as substrates.

### 8. Immunocytochemical Staining

Immunocytochemical staining was performed as the same method described elsewhere (Kawai et al. (1986) Pediatr. Res. 20, 915-919) with minor modifications. E5 cells were plated on sterile coverslips 24 hr. prior to staining. Coverslips were washed with PBS and fixed with ice-cold 95% ethanol in PBS. Fixed coverslips were again washed with PBS, and incubated for 1 h. at room temperature with 3% BSA in TBS, and then reacted for another 1 h. with the 1:400 diluted absorbed $AS_{E5}$ (described above) in 3% BSA in TBS. The coverslips were then washed with 0.05% Nonidet P-40 (NP-40) in 3% BSA in TBS three times, and overlaid with 1:50 diluted fluorescein isothiocyanate (FITC)-conjugated goat anti-rabbit IgG (J.D. Biologicals, Downsview, Ontario, Canada). After a 30 minute incubation, the specimens were washed in the same way, and mounted on glass microscope slides by using 50% (v/v) glycerol in PBS. Fluorescence microscopy was done with a Zeiss microscope.

### 9. Analysis of Peptide N-Glycosidase F Treated Membrane Components

Deglycosylation experiments were performed with peptide N-glycosidase F (PNGase F, Boehringer Mannheim Canada, Dorval, Quebec, Canada) using a modification of the method described by Tarentino et al. (1985) Biochemistry 24, 4665-4671. Digestion was preformed in a final volume of 100 $\mu$l consisting of the following ingredients added in order, 100 $\mu$g of purified plasma membrane vesicles from R1.1 or E5 cells, 1.25% NP-40, 0.5% 2-mercaptoethanol, 2mM PMSF, 30 $\mu$M leupeptin, 4 $\mu$g/ml pepstatin, 2 U of PNGase F, 0.05 M sodium phosphate buffer (pH 8.6) 0.075% BSA, and 0.2% SDS. Incubation was done for 24 h. at 37°C. Pretreatment control samples were processed in the same manner except PNGase F was not included. Thereafter, the digested membrane proteins were divided into two parts, and each was subjected to SDS-PAGE and immunostaining as described above.

### 10. Immunofluorescence Studies

Exponentially growing cells were washed three times with PBS, counted and adjusted to 1 x 10^5 /ml of PBS containing 1% BSA. Cells were incubated with 10 $\mu$l of FITC-conjugated goat anti-Thy 1.2 (Becton-Dickinson, Mountain View, CA) for 1 hr. at room temperature and then washed with PBS three times. Membrane fluorescence of treated cells was observed using a Zeiss microscope and the percentage of stained cells were estimated after counting more than 250 cells. Expression of other T cell surface antigens, Lyt-2 and L3T4, were evaluated using a two parameter fluorescence distribution analysis. Cells were prepared as described above and stained simultaneously with 5 $\mu$l of FITC-conjugated anti-mouse Lyt 2 monoclonal antibody (Becton-Dickinson) and 4 $\mu$l of phycoerythrin-conjugated anti-mouse L3T4 monoclonal antibody (Becton-Dickinson). After washing, the specimens were analyzed using a fluorescence-activated cell sorter (FACScan, Becton-Dickinson). Control cells were treated in the same manner except the fluorescent dyes were excluded.

**Claims**

1. A plasma membrane antigen having a molecular weight of approximately 200 kDa, the antigen being correlated with resistance to platinum-containing anti-neoplastic agents.

2. The plasma membrane antigen as claimed in claim 1 correlated with resistance to cis-diamminedichloroplatinum (II) (CDDP).

3. The plasma membrane antigen as claimed in claim 1 correlated with resistance to carboplatin, tetraplatin or iproplatin.

4. The plasma membran antigen as claimed in any one of claims 1 to 3, which is the plasma membrane glycoprotein $CP^R$-200.

5. A monoclonal antibody specific to a plasma membrane antigen having a molecular weight of approximately 200 kDa and being correlated with resistance to platinum-containing antineoplastic agents.

6. The monoclonal antibody as claimed in claim 5, wherein the plasma membrane antigen is correlated with resistance to cis-diamminedichloroplatinum (II) (CDDP).

7. The monoclonal antibody as claimed in claim 5, wherein the plasma membrane antigen is correlated with resistance to carboplatin, tetraplatin or iproplatin.

8. The monoclonal antibody as claimed in any one of claims 5 to 7, wherein the plasma membrane antigen is $CP^R$-200.

9. A nucleotide sequence encoding a plasma membrane antigen having a molecular weight of approximately 200 kDa and being correlated with resistance to platinum-containing antineoplastic agents.

10. The nucleotide sequence as claimed in claim 9, wherein the antigen is $CP^R$-200.

11. The nucleotide sequence as claimed in claims 9 or 10, wherein the sequence is a DNA sequence.

12. The nucleotide sequence as claimed in claim 11 or a fragment thereof having at least 50 nucleotide bases, said sequence being joined to a non-wild type DNA to form a hybrid DNA.

13. The sequence as claimed in claim 12, wherein the non-wild type DNA comprises a replication system recognized by a unicellular microorganism.

14. An expression vector comprising a nucleotide sequence as claimed in any one of claims 9 to 13.

15. A nucleotide sequence having sufficient homology with the sequence as claimed in claims 9 to 13 to enable it to function as a probe.

16. A nucleotide sequence having at least 60% homology with the sequence of claims 9 to 13 over a span of at least 50 nucleotide bases as measured by hybridization therewith.

17. A cell containing a functional expression vector capable of replication in the cell, the vector comprising transcriptional and translational signals functional in the cell and a DNA sequence encoding a plasma membrane antigen having a molecular weight of approximately 200 kDa and being correlated with resistance to platinum-containing antineoplastic agents or fragment thereof and being under the transcriptional and translational control of said signals.

18. The cell as claimed in claim 17, wherein the sequence is a cDNA sequence.

19. The use of a monoclonal antibody as claimed in any one of claims 5 to 8 for detection and diagnosis of cells resistant to platinum-containing anti-neoplastic agents.

20. The use of a nucleotide sequence as claimed in claims 15 or 16 for detection and diagnosis of cells

9

resistant to platinum-containing anti-neoplastic agents.

21. A kit containing at least one monoclonal antibody as claimed in any one of claims 5 to 8.

22. A kit containing at least one nucleotide sequence as claimed in claims 15 or 16.

FIG. 1

FIG. 2

FIG.3

FIG.4

FIG.5

FIG.6

FIG.7